# EUROPEAN PATENT APPLICATION

(11) **EP 4 631 479 A1**
(43) Date of publication of application: **15.10.2025**
(21) Application number: 23917748.8
(22) Date of filing: 22.12.2023
(51) Int. Cl.: A61F 2/78

(54) **PROSTHETIC SOCKET**

(30) Priority: 16.01.2023 JP 2023004614
(71) Applicant: The University of Electro-Communications, Chofu-shi, Tokyo 182-8585 (JP)
(72) Inventor: YOKOI, Hiroshi, Chofu-shi, Tokyo 182-8585 (JP); INOUE, Yuki, Chofu-shi, Tokyo 182-8585 (JP); YAMANOI, Yusuke, Chofu-shi, Tokyo 182-8585 (JP); YABUKI, Yoshiko, Chofu-shi, Tokyo 182-8585 (JP); KURODA, Yuki, Chofu-shi, Tokyo 182-8585 (JP)
(74) Representative: Novaimo
(86) International application number: PCT/JP2023/046121
(87) International publication number: WO 2024/154538

(57) **Abstract**

Provided is a prosthetic socket capable of realizing both a decrease in pain felt by a wearer and generation of sufficient fastening force. A socket body 10 capable of forming a tubular shape that covers an outer periphery of a living body end portion FA includes an inner peripheral end portion 14 and an outer peripheral end portion 15. The outer peripheral end portion 15 overlaps the inner peripheral end portion 14 from an outer side in a radial direction of the tubular shape, and by increasing or decreasing an overlap area in the circumferential direction between the inner peripheral end portion 14 and the outer peripheral end portion 15, a total circumferential length of the tubular shape is decreased or increased. A plurality of through holes 21 are passed through the inner peripheral end portion 14, and are arranged in an axial direction of the tubular shape and spaced apart from each other. A plurality of linear bodies 20 include a one end portion 20A that is supported by the outer peripheral end portion 15 on one side in the circumferential direction of a tip 14A of the inner peripheral end portion 14, and an other end portion 20B that is supported by the socket body 10 on another side in the circumferential direction of the tip 14A of the inner peripheral end portion 14, the plurality of linear bodies 20 being passed through the through holes 21, being arranged in the axial direction and spaced apart from each other.

## Description

### [Technical Field]

The present invention relates to prosthetic sockets.

### [Background Art]

Patients having hypoplasia or patients having lost a part of their four limbs, i.e., upper limb or lower limb, due to congenital causes or acquired causes such as accidents may wear prosthesis so as to restore the shapes and functions of the four limbs. A common prosthesis is composed of a prosthetic socket that is attached to and held by a living body end portion, and an upper or a lower extremity tool that is mounted on the prosthetic socket.

As one example of a conventional prosthetic socket, a prosthetic socket equipped with two shell-shaped members, an inner shell-shaped member and an outer shell-shaped member, that are curved in an arc shape is known. A tubular shape that covers an outer periphery of a living body end portion is formed by having both end portions of the outer shell-shaped member overlap both end portions of the inner shell-shaped member from an outer side in a radial direction of the tubular shape. A total circumferential length of the tubular shape is decreased or increased by increasing or decreasing an overlap area in the circumferential direction between both end portions of the inner shell-shaped member and both end portions of the outer shell-shaped member.

### [Citation List]

### [Patent Literature]

[Patent Document 1] Japanese Translation of PCT International Application Publication No. JP-T-2019-51484

### [Summary of Invention]

### [Problem to be Solved by the Invention]

In order to firmly attach the conventional prosthetic socket described above to the living body end portion, it is necessary to tighten the two shell-shaped members that cover the circumference of the living body end portion, and to generate a sufficient fastening force. By tightening the two shell-shaped members and decreasing the total circumferential length of the tubular shape to be shorter than a circumferential length of the living body end portion, pressure is generated between inner circumferential surfaces of the two shell-shaped members and a skin surface of the living body end portion, by which the inner circumferential surface of the two shell-shaped members and the skin surface of the living body end portion are adhered closely against each other.

However, both ends of the inner shell-shaped member form a level difference in a radial direction with the outer shell-shaped member, and a gap is formed between the outer shell-shaped member and the skin surface of the living body end portion that are arranged adjacent the level difference. An excess portion of the skin surface of the living body end portion will converge in the gap formed by the level difference by an operation to squeeze the prosthetic socket thereon, by which the skin surface will be folded and creased, possibly causing the wearer to feel pain. Therefore, there is a demand for a prosthetic socket that realizes both a decrease in pain felt by the wearer and the generation of sufficient fastening force. Especially in the case of infants, prosthetic sockets that cause pain are not practical, and there are high demands for a prosthetic socket that may generate sufficient fastening force without causing pain.

In view of the problems of the prior art described above, the present invention aims at providing a prosthetic socket capable of realizing both a decrease in pain felt by a wearer and generation of sufficient fastening force.

### [Means to Solve the Problem]

In order to solve the problems described above, one typical prosthetic socket according to the present invention includes:
a socket body in which one or a plurality of members may form a tubular shape configured to cover an outer periphery of a living body end portion, the socket body including at least one pair of an inner peripheral end portion and an outer peripheral end portion, the outer peripheral end portion overlapping the inner peripheral end portion from an outer side in a radial direction of the tubular shape, and an overlap area in a circumferential direction between the inner peripheral end portion and the outer peripheral end portion being increased or decreased to decrease or increase a total circumferential length of the tubular shape;
a plurality of through holes being configured to pass through the inner peripheral end portion, the plurality of through holes being arranged in an axial direction of the tubular shape and spaced apart from each other; and
a plurality of linear bodies including one end portion supported by the outer peripheral end portion at one side in the circumferential direction than a tip of the inner peripheral end portion, and an other end portion supported by the socket body at the other side in the circumferential direction than the tip of the inner peripheral end portion, the linear bodies being configured to pass through the through holes between the one end portion and the other end portion, being arranged in the axial direction and spaced apart from each other.

### [Effects of Invention]

According to the present invention, a prosthetic socket capable of realizing both a decrease in pain felt by a wearer and generation of sufficient fastening force may be provided.

Problems, configurations, and effects other than those described above will become apparent from the following description of the embodiments.

### [Brief Description of Drawings]

FIG. 1 is a side view of a prosthetic socket according to a first embodiment of a present invention.
FIG. 2 is a cross-sectional view taken at arrow II-II of the prosthetic socket of FIG. 1.
FIG. 3 is a perspective view in which a plurality of through holes and a plurality of linear bodies are illustrated together with the socket body.
FIG. 4 is a cross-sectional view of the prosthetic socket in a fastened state.
FIG. 5 is a side view of the prosthetic socket according to a second embodiment of the present invention.
FIG. 6 is a cross-sectional view of a prosthetic socket according to a third embodiment illustrating a state prior to being attached to a forearm portion.
FIG. 7 is a cross-sectional view of the prosthetic socket according to the third embodiment, illustrating a state where the prosthetic socket is attached to the forearm portion.

### [Description of Embodiments]

### (First Embodiment)

A first embodiment according to the present invention will be described in detail with reference to the drawings. The present embodiment illustrates an example in which the present invention has been applied to a prosthetic socket constituting a prosthetic hand, but the present invention may also be applied to a prosthetic socket constituting a prosthetic leg.

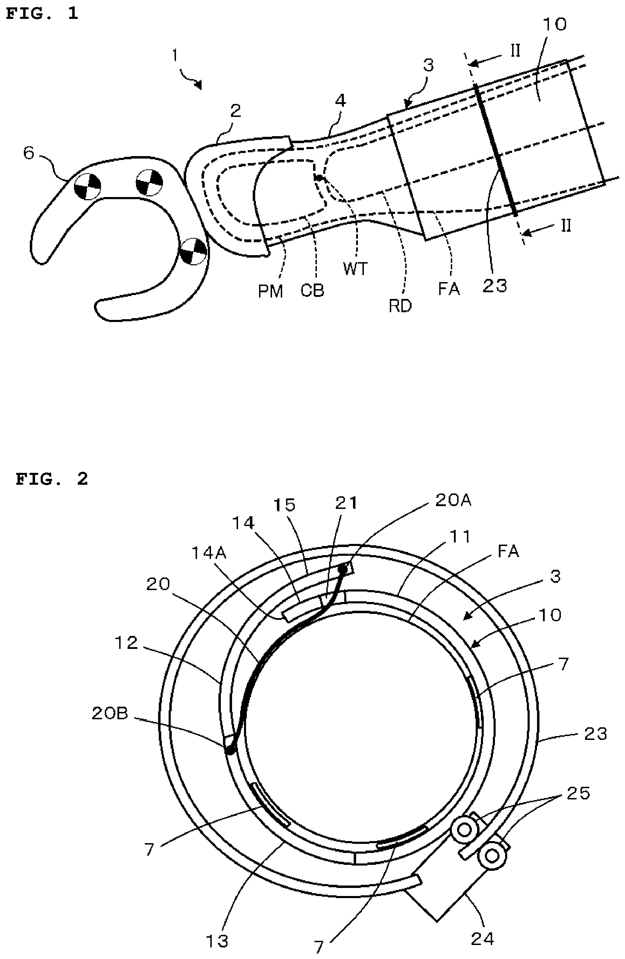

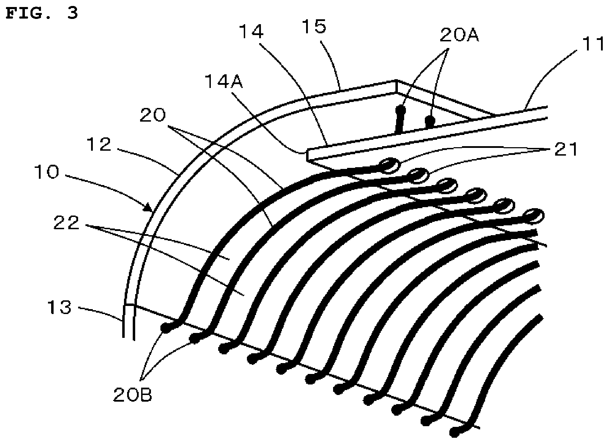

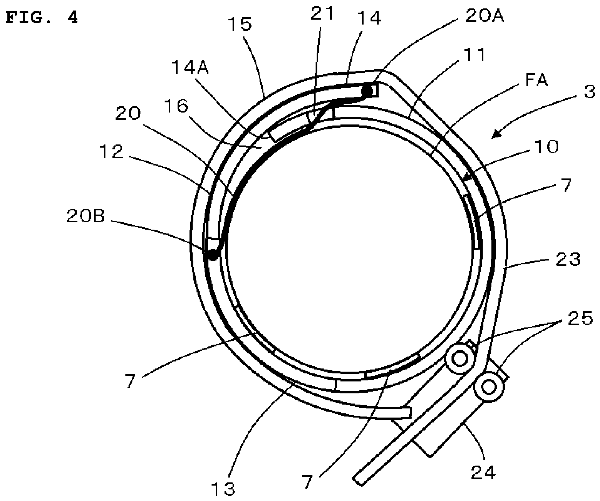

FIG. 1 is a side view illustrating a state in which a forearm portion socket 3 serving as a prosthetic socket according to the present embodiment is attached to a forearm portion FA serving as a living body end portion, FIG. 2 is a cross-sectional view taken at arrow II-II of the forearm portion socket 3 of FIG. 1, FIG. 3 is a perspective view illustrating a plurality of through holes 21 and a plurality of linear bodies 20 together with a socket body 10, and FIG. 4 is a cross-sectional view of the forearm portion socket 3 in a tightened state.

As illustrated in FIG. 1, a rear end portion of a carpal bone CB of a palm portion PM is connected via a wrist, i.e., radiocarpal articulation, WT to a front end portion of a radius RD of the forearm portion FA. The palm portion PM tilts in an up-down direction, i.e., dorsal flexion direction and palmar flexion direction, about the wrist WT with respect to the forearm portion FA. Further, the palm portion PM tilts in a right-left direction, i.e., radial flexion direction and ulnar flexion direction, about the wrist WT with respect to the forearm portion FA. That is, the palm portion PM tilts via the wrist WT with respect to the forearm portion FA. In FIG. 1, a center of rotation of the palm portion PM with respect to the forearm portion FA is illustrated schematically as the wrist WT.

A prosthetic hand 1 includes a palm portion socket 2, the forearm portion socket 3, an intermediate cover 4, an articulation mechanism (not shown), and an upper extremity tool 6. The prosthetic hand 1 is a myoelectric hand that may detect a bioelectric potential signal, such as a minute current, i.e., surface myogenic potential, that is generated when a muscle contracts, via an electrode 7 (illustrated in FIG. 2), drive an actuator, such as an electric motor, being built therein based on the detected bioelectric potential signal, and drive the articulation mechanism or the upper extremity tool 6 to realize a gripping operation. The prosthetic hand 1 may also be a prosthetic hand other than the myoelectric hand, such as a body-powered prosthetic hand.

The palm portion socket 2 is formed in a tubular shape with a bottom by one or a plurality of members, and in a state where at least the front end portion of the palm portion PM is covered, it is fixed to the palm portion socket 2 so as not to move in the front-rear direction and in the rotational direction.

The forearm portion socket 3 includes the socket body 10 that may be formed to have a tubular shape. In a state where an outer circumferential surface of the forearm portion FA is covered, the socket body 10 is fixed to the forearm portion FA so as not to move in the front-rear direction and the rotational direction. The details of the forearm portion socket 3 will be described later.

The intermediate cover 4 is formed in a tubular shape by one or a plurality of members, and covers the rear end portion of the palm portion PM, the front end portion of the forearm portion FA, and the wrist WT between the palm portion socket 2 and the forearm portion socket 3.

The articulation mechanism connects the palm portion socket 2 and the forearm portion socket 3, and tilts the palm portion PM in the dorsal flexion direction, the palmar flexion direction, the radial flexion direction, and the ulnar flexion direction.

As illustrated in FIGs. 2 and 3, the forearm portion socket 3 includes the socket body 10 and the plurality of linear bodies 20.

The socket body 10 is one member configured to enable a tubular shape to be formed by a plurality of, which according to the illustrated example is three, shell-shaped portions, which are an inner shell-shaped portion 11, an outer shell-shaped portion 12, and an intermediate shell-shaped portion 13. Each shell-shaped portion 11, 12, and 13 has a plate shape that curves in an arc shape, and for example, it is formed of a rigid resin having flexibility. One edge portion of the inner shell-shaped portion 11 is connected in a flexible manner to one edge portion of the intermediate shell-shaped portion 13, and one edge portion of the outer shell-shaped portion 12 is connected in a flexible manner to the other edge portion of the intermediate shell-shaped portion 13. In the following description, a circumferential direction of the tubular shape formed by the socket body 10 is referred to as a tubular circumferential direction, a radial direction thereof is referred to as a tubular radial direction, and an axial direction thereof, i.e., direction along a center axis, is referred to as a tubular shaft direction.

A plurality of, which according to the illustrated example is three, electrodes 7 are spaced apart from each other in the tubular circumferential direction and fixed to the inner circumferential surface of the socket body 10, which in the illustrated example is the inner circumferential surface of the inner shell-shaped portion 11 and the inner circumferential surface of the intermediate shell-shaped portion 13. The electrode 7 must be adhered closely to the skin surface stably with an appropriate pressure. Further, the electrode 7 must also be provided with a holding force to fix the socket body 10 to the skin surface. Therefore, it is preferable to form the electrode 7 by a material, such as rubber, that generates a strong shearing force by frictional force with respect to the skin surface.

The other end portion of the inner shell-shaped portion 11 constitutes an inner peripheral end portion 14 and the other end portion of the outer shell-shaped portion 12 constitutes an outer peripheral end portion 15. The inner peripheral end portion 14 and the outer peripheral end portion 15 are separated in a relatively movable manner in the tubular circumferential direction and the tubular radial direction. The outer peripheral end portion 15 overlaps from the outer side in the tubular radial direction to the inner peripheral end portion 14, and by the increase or decrease of overlap area between the inner peripheral end portion 14 and the outer peripheral end portion 15 in the tubular circumferential direction, the total circumferential length of the tubular shape decreases or increases.

As illustrated in FIG. 3, the plurality of through holes 21 are formed on the inner peripheral end portion 14. The plurality of through holes 21 are arranged across approximately an entire area in the tubular shaft direction of the inner peripheral end portion 14, and are arranged at equal intervals in the tubular shaft direction and spaced apart from each other, and are passed through the inner peripheral end portion 14. The interval of the through holes 21 arranged along the tubular shaft direction may be narrow within a machinable range, and for example, may be around 50 mm, but specifically, they are preferably 1 mm or more and 20 mm or less, and in the illustrated example, it is approximately 3 mm.

A linear body 20 is a filament body formed for example of soft resin, such as nylon resin, and the cross-sectional shape thereof may be a rectangle, an ellipse, or a circle, and preferably a circle. A diameter of the linear body 20, which is a diameter in the case of a circular cross-section, which is a long diameter in the case of an elliptic cross-section, which is the length of a side in the case of a square cross-section, and is the length of a long side in the case of a rectangular cross-section, is preferably 0.5 mm or more and 3 mm or less, and in the illustrated example, it is approximately 1 mm. One end portion 20A of the linear body 20 is connected to and supported by the outer peripheral end portion 15 on one side in the tubular circumferential direction than a tip 14A of the inner peripheral end portion 14. An other end portion 20B of the linear body 20 is connected to and supported by the socket body 10, which in the case of the illustrated example is the intermediate shell-shaped portion 13, on the other side in the tubular circumferential direction than the tip 14A of the inner peripheral end portion 14. The plurality of linear bodies 20 are respectively passed through the through holes 21 between the one end portion 20A and the other end portion 20B, and are arranged at equal intervals in the tubular shaft direction and spaced apart from each other. As described, the plurality of linear bodies 20 are arranged approximately in parallel in a comb tooth-like manner along the tubular shaft direction, and forms a plurality of fine slits 22 between mutually adjacent linear bodies 20. The plurality of slits 22 are arranged along the tubular shaft direction between the tip 14A of the inner peripheral end portion 14 and the other end portion 20B of the linear body 20, and the respective slits 22 are extended in an elongated shape in the tubular circumferential direction.

The through holes 21 move smoothly between the one end portion 20A and the other end portion 20B of the linear body 20 in response to the increase or decrease of the total circumferential length of the tubular shape. For example, in a case where the overlap area in the tubular circumferential direction between the inner peripheral end portion 14 and the outer peripheral end portion 15 increases and the total circumferential length of the tubular shape decreases, the tip 14A and the through holes 21 of the inner peripheral end portion 14 move toward the other end portion 20B of the linear body 20, and the slits 22 shrink. In contrast, in a case where the overlap area in the tubular circumferential direction between the inner peripheral end portion 14 and the outer peripheral end portion 15 decreases and the total circumferential length of the tubular shape increases, the tip 14A and the through holes 21 of the inner peripheral end portion 14 move toward the one end portion 20A of the linear body 20, and the slits 22 expand.

A linear or belt-like tightening member, such as a wire, 23 for fastening the socket body 10 is wound around the outer periphery of the socket body 10. A fastening device 24 is connected to one end of the tightening member 23, and the other end side of the tightening member 23 is nipped between a pair of fastening rollers 25 of the fastening device 24. By driving the fastening rollers 25 and shrinking the total circumferential length of the tightening member 23, as illustrated in FIG. 4, the socket body 10 is fastened by the tightening member 23, and the inner circumferential surface of the socket body 10 and the electrode 7 adhere closely to the skin surface of the forearm portion FA. The number of the set of the tightening member 23 and the fastening device 24 that fasten the outer periphery of the socket body is not limited to one set, and there may be a plurality of sets.

When attaching the forearm portion socket 3 to the forearm portion FA, as illustrated in FIG. 2, the tightening member 23 is loosened, and the tubular shape of the socket body 10 is expanded to cover the outer periphery of the forearm portion FA with the socket body 10. In this state, between the through holes 21 and the other end portion 20B of the linear body 20, the plurality of linear bodies 20 are arranged in the tubular shaft direction between the inner circumferential surface of the socket body 10 and the skin surface of the forearm portion FA, and the plurality of slits 22 are arranged in the tubular shaft direction (refer to FIG. 3).

Next, in a state where the outer periphery of the forearm portion FA is covered by the socket body 10, the fastening device 24 is driven, and the outer periphery of the socket body 10 is fastened by the tightening member 23. When the socket body 10 is fastened, as illustrated in FIG. 4, the inner circumferential surface of the socket body 10, the electrode 7, and the plurality of linear bodies 20 come into contact with the skin surface of the forearm portion FA. In this state, the tip 14A of the inner peripheral end portion 14 forms a level difference between the outer shell-shaped portion 12, such that a gap, or excess space, 16 is formed between the outer shell-shaped portion 12 and the skin surface of the forearm portion FA.

By the squeezing operation of the socket body 10, the skin surface of the forearm portion FA is pulled by the inner circumferential surface of the socket body 10 and the electrode 7, such that the excess portion of the skin surface tends to be accumulated in the gap 16 formed by the level difference, but the entry of the excess portion of the skin surface into the gap 16 is prevented by the plurality of linear bodies 20, and the excess portion of skin surface moves smoothly on the linear body 20 in the tubular circumferential direction, dispersed into and absorbed by the plurality of fine slits 22. Therefore, the excess portion of the skin surface will not be folded and creased, such that the pain felt by the wearer may be reduced while generating a sufficient fastening force.

### (Second Embodiment)

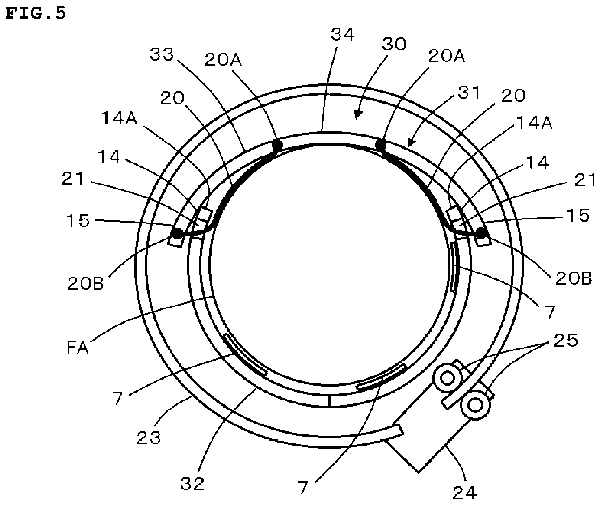

Next, a second embodiment according to the present invention will be described with reference to FIG. 5. FIG. 5 is a side view illustrating a state in which a forearm portion socket 30 according to the second embodiment is attached to a forearm portion FA. Similar configurations as the first embodiment are denoted with the same reference numbers, and detailed descriptions thereof are omitted.

In the first embodiment, the socket body 10 is composed of one member, but it is possible to have the socket body composed of a plurality of members. The second embodiment illustrates a configuration where a socket body 31 is formed of two members, which are an inner shell-shaped member 32 and an outer shell-shaped member 33. The inner shell-shaped member 32 and the outer shell-shaped member 33 are plate shaped and curved in an arc shape, and they are formed of rigid resin having flexibility, for example.

The inner shell-shaped member 32 includes inner peripheral end portions 14 at both ends in the tubular circumferential direction. The outer shell-shaped member 33 includes outer peripheral end portions 15 at both ends in the tubular circumferential direction, and an outer intermediate portion 34 disposed between the outer peripheral end portions 15. The outer peripheral end portions 15 overlap the inner peripheral end portion 14 from the outer side in the tubular radial direction, and the outer intermediate portion 34 does not overlap the inner shell-shaped member 32. By the increase or decrease of the overlap areas of the inner peripheral end portions 14 and the outer peripheral end portions 15 in the tubular circumferential direction, the total circumferential length of the tubular shape decreases or increases. As described, the socket body 31 includes two pairs of inner peripheral end portions 14 and outer peripheral end portions 15.

A plurality of through holes 21 are formed at the two locations of the inner peripheral end portions 14, and the plurality of linear bodies 20 are disposed to correspond to each pair of the inner peripheral end portion 14 and the outer peripheral end portion 15. In each pair of the inner peripheral end portion 14 and the outer peripheral end portion 15, the linear bodies 20 are passed through the through holes 21, wherein the one end portion 20A of the linear bodies 20 is connected to and supported by the outer peripheral end portion 15, and the other end portion 20B of the linear body 20 is connected to and supported by the outer intermediate portion 34. By utilizing the forearm portion socket 30, similar to the first embodiment, the pain felt by the wearer may be reduced while generating a sufficient fastening force.

### (Third Embodiment)

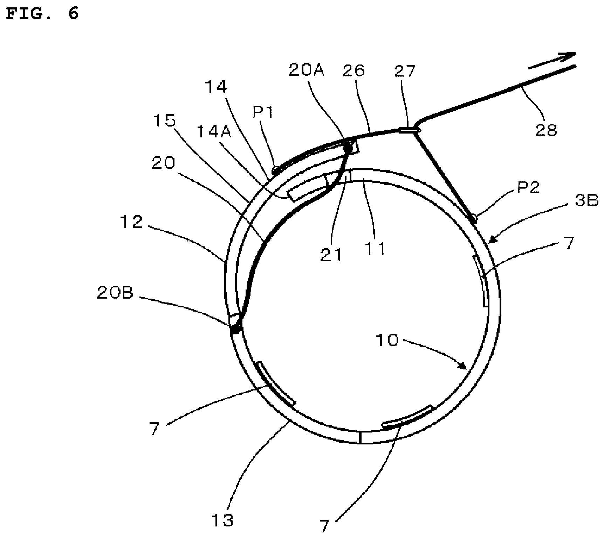

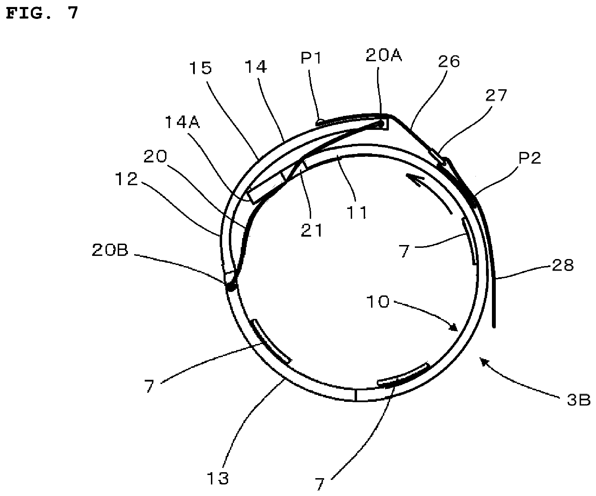

Next, a third embodiment according to the present invention will be described with reference to FIGs. 6 and 7. FIGs. 6 and 7 are a cross-sectional view of a forearm portion socket 3B according to the third embodiment. Similar configurations as the first embodiment are denoted with the same reference numbers, and detailed descriptions thereof are omitted.

According to the first embodiment, for example, by electrically driving the fastening device 24, the socket body 10 may be fastened by the tightening member 23 so as to have the inner side of the socket body 10 come into contact with the skin surface of the forearm portion. However, an equipment for feeding power to the fastening device 24 must be provided, and the increase in weight of the fastening device 24 may be a burden on the wearer. The present embodiment solves such problems.

In FIG. 6 illustrating the state prior to attaching the socket body 10, one end of a belt 26 is fixed by a screw or a rivet to an outer circumferential surface, i.e., point P1, of the outer shell-shaped portion 12. The belt 26 extends beyond a terminal end of the outer peripheral end portion 15, and the other end thereof is connected to a rectangular link 27 made of metal. Meanwhile, one end of a hook-and-loop fastener 28 is fixed by a screw or a rivet to an outer circumferential surface, i.e., point P2, of the inner shell-shaped portion 11. The other end of the hook-and-loop fastener 28 is passed through an inner side of the rectangular link 27 made of metal and drawn out, where the hook-and-loop fastener 28 is folded back at the rectangular link 27 and extended in the opposite direction. In the present embodiment, the belt 26, the rectangular link 27, and the hook-and-loop fastener 28 constitute a fastening device that fastens the outer periphery of the socket body 10.

When attaching the forearm portion socket 3B to the forearm portion (not shown) of the wearer, the socket body 10 illustrated in FIG. 6 is first arranged on the periphery of the forearm portion, and thereafter, the wearer holds the end portion of the hook-and-loop fastener 28 and pulls the end portion to an opposite direction as the belt 26. Thereby, as illustrated in FIG.7, the distance between one end, i.e., point P2, of the hook-and-loop fastener 28 and the rectangular link 27 is reduced, such that the point P1 of the outer circumferential surface of the outer shell-shaped portion 12 and the point P2 of the outer circumferential surface of the inner shell-shaped portion 11 approach each other, and the socket body 10 shrinks in diameter. Based on a principle of a movable pulley, a tensile force applied to the rectangular link 27 is approximately twice the tensile force of the hook-and-loop fastener 28, such that even if the wearer is a child having a small physical strength, he/she may fasten the socket body 10 easily without asking for any support from a helper, and the inner side of the socket body 10 may be made to come into contact appropriately with the skin surface of the forearm portion.

After having the socket body 10 and forearm portion come into contact with an appropriate surface pressure, as illustrated in FIG. 7, the hook-and-loop fastener 28 that has been folded back is overlapped with itself, and the overlapped surfaces are mutually connected, such that the tension of the belt 26 and the hook-and-loop fastener 28 applied between point P1 and point P2 may be supported. Therefore, the socket body 10 may be suppressed from expanding in diameter, such that the forearm portion socket 3B may be prevented from falling from the forearm portion, and the electrode 7 adhered closely to the skin surface may detect the bioelectric potential signal appropriately.

Alternatively, instead of the hook-and-loop fastener, any belt-like member that may be passed through the rectangular link 27 and then folded back to support the tension applied between the fixed end portion and the folded position may be used.

The present invention is not limited to the embodiments described above, and may include various modifications thereof. For example, the above-described embodiments are illustrated in detail to facilitate understanding of the invention, and the present invention is not limited to having all the components illustrated in the embodiments. A portion of the components of an embodiment may be replaced with the components of other embodiments, or a component of an embodiment may be added to the components of other embodiments. Further, a portion of the components of the respective embodiments may be added to, deleted from, or replaced with the components of other embodiments.

The present specification includes the following disclosures of the invention.

### (First Aspect)

A prosthetic socket including:
a socket body in which one or a plurality of members may form a tubular shape configured to cover an outer periphery of a living body end portion, the socket body including at least one pair of an inner peripheral end portion and an outer peripheral end portion, the outer peripheral end portion overlapping the inner peripheral end portion from an outer side in a radial direction of the tubular shape, and an overlap area in a circumferential direction between the inner peripheral end portion and the outer peripheral end portion being increased or decreased to decrease or increase a total circumferential length of the tubular shape;
a plurality of through holes being configured to pass through the inner peripheral end portion, the plurality of through holes being arranged in an axial direction of the tubular shape and spaced apart from each other; and
a plurality of linear bodies including one end portion supported by the outer peripheral end portion at one side in the circumferential direction than a tip of the inner peripheral end portion, and an other end portion supported by the socket body at the other side in the circumferential direction than the tip of the inner peripheral end portion, the linear bodies being configured to pass through the through holes between the one end portion and the other end portion, being arranged in the axial direction and spaced apart from each other.

### (Second Aspect)

The prosthetic socket according to the first aspect,
wherein the linear body is formed of soft resin.

### (Third Aspect)

The prosthetic socket according to the first or second aspect,
wherein an interval between the through holes along the axial direction is 1 mm or more and 20 mm or less, and
wherein a diameter of the linear body is 0.5 mm or more and 3 mm or less.

The prosthetic socket according to any of the first to third aspects, further including
a fastening device configured to fasten an outer periphery of the socket body.

The prosthetic socket according to the fourth aspect,
wherein the fastening device includes
   a belt having one end fixed to an outer circumferential surface of the socket body in a vicinity of the outer peripheral end portion, the belt being configured to extend beyond the outer peripheral end portion,
   a link being fixed to an other end of the belt, and
   a hook-and-loop fastener having one end fixed to the outer circumferential surface of the socket body, the hook-and-loop fastener being passed through the link and folded back, and
wherein the outer periphery of the socket body is fastened by a tension of the belt and the hook-and-loop fastener.

### [Reference Signs List]

1: prosthetic hand, 2: palm portion socket, 3, 30, 3B: forearm portion socket (prosthetic socket), 4: intermediate cover, 6: upper extremity tool, 7: electrode, 10, 31: socket body, 11: inner shell-shaped portion, 12: outer shell-shaped portion, 13: intermediate shell-shaped portion, 14: inner peripheral end portion, 14A: tip of inner peripheral end portion, 15: outer peripheral end portion, 16: gap, 20: linear body, 20A: one end portion of linear body, 20B: the other end portion of linear body, 21: through hole, 22: slit, 23: tightening member, 24: fastening device, 25: fastening roller, 26: belt, 27: rectangular link, 28: hook-and-loop fastener, 32: inner shell-shaped member, 33: outer shell-shaped member, 34: outer intermediate portion, PM: palm portion, WT: wrist, FA: forearm portion (living body end portion), CB: carpal bone, RD: radius

## Claims

1. A prosthetic socket comprising:
a socket body in which one or a plurality of members may form a tubular shape configured to cover an outer periphery of a living body end portion, the socket body including at least one pair of an inner peripheral end portion and an outer peripheral end portion, the outer peripheral end portion overlapping the inner peripheral end portion from an outer side in a radial direction of the tubular shape, and an overlap area in a circumferential direction between the inner peripheral end portion and the outer peripheral end portion being increased or decreased to decrease or increase a total circumferential length of the tubular shape;
a plurality of through holes being configured to pass through the inner peripheral end portion, the plurality of through holes being arranged in an axial direction of the tubular shape and spaced apart from each other; and
a plurality of linear bodies including one end portion supported by the outer peripheral end portion at one side in the circumferential direction than a tip of the inner peripheral end portion, and an other end portion supported by the socket body at the other side in the circumferential direction than the tip of the inner peripheral end portion, the linear bodies being configured to pass through the through holes between the one end portion and the other end portion, being arranged in the axial direction and spaced apart from each other.

2. The prosthetic socket according to claim 1,
wherein the linear body is formed of soft resin.

3. The prosthetic socket according to claim 1,
wherein an interval between the through holes along the axial direction is 1 mm or more and 20 mm or less, and
wherein a diameter of the linear body is 0.5 mm or more and 3 mm or less.

4. The prosthetic socket according to any one of claims 1 to 3, further comprising
a fastening device configured to fasten an outer periphery of the socket body.

5. The prosthetic socket according to claim 4,
wherein the fastening device includes
a belt having one end fixed to an outer circumferential surface of the socket body in a vicinity of the outer peripheral end portion, the belt being configured to extend beyond the outer peripheral end portion,
a link being fixed to an other end of the belt, and
a hook-and-loop fastener having one end fixed to the outer circumferential surface of the socket body, the hook-and-loop fastener being passed through the link and folded back, and
wherein the outer periphery of the socket body is fastened by a tension of the belt and the hook-and-loop fastener.
